(19) Europäisches Patentamt / European Patent Office / Office européen des brevets

(11) **EP 2 669 005 B1**

(12) **FASCICULE DE BREVET EUROPEEN**

(45) Date de publication et mention
de la délivrance du brevet:
**29.08.2018 Bulletin 2018/35**

(51) Int Cl.:
*B01J 23/44* (2006.01)       *B01J 23/50* (2006.01)
*B01J 35/00* (2006.01)       *B01J 37/02* (2006.01)
*C10G 45/40* (2006.01)       *C07C 7/167* (2006.01)

(21) Numéro de dépôt: **13305467.6**

(22) Date de dépôt: **10.04.2013**

(54) **Procédé de préparation d'un catalyseur comprenant du palladium et de l'argent utile en hydrogenation selective**

Herstellungsverfahren eines für selektives Hydrierverfahren verwendbaren Katalysators, der Palladium und Silber enthält

Preparation process of a catalyst comprising palladium and silver usable in selective hydrogenation

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.05.2012 FR 1201572**

(43) Date de publication de la demande:
**04.12.2013 Bulletin 2013/49**

(73) Titulaire: **IFP Energies nouvelles
92500 Rueil-Malmaison (FR)**

(72) Inventeurs:
• **CABIAC, Amandine
69700 GIVORS (FR)**
• **ZOZAYA, Vincent
69360 SAINT-SYMPHORIEN-D'OZON (FR)**
• **CHAMBARD, Alexandre
24300 JAVERLHAC (FR)**
• **THOMAZEAU, Cecile
69008 LYON (FR)**

(56) Documents cités:
EP-A1- 0 780 155       FR-A1- 2 720 956
FR-A1- 2 882 531       FR-A1- 2 963 344
US-A1- 2010 197 488

**Description**

Le procédé d'hydrogénation sélective permet de transformer les composés polyinsaturés des coupes pétrolières par conversion des composés les plus insaturés vers les alcènes correspondants en évitant la saturation totale et donc la formation des alcanes correspondants.

L'objet de l'invention est de proposer un catalyseur ainsi qu'un mode de préparation de ce catalyseur. Ce catalyseur possède des très bonnes performances pour les procédés d'hydrogénation sélective des composés hydrocarbonés insaturés présents dans les coupes d'hydrocarbures, de préférence des coupes issues du vapocraquage et/ou du craquage catalytique et en particulier les coupes C3.

**ART ANTÉRIEUR**

Les catalyseurs d'hydrogénation sélective de ces coupes sont souvent à base de palladium, sous forme de petites particules métalliques déposées sur un support qui peut être un oxyde réfractaire sous forme de billes, d'extrudés, de trilobes ou sous des formes présentant d'autres géométries. La teneur en palladium et la taille des particules de palladium font partie des critères qui ont une importance sur l'activité et la sélectivité des catalyseurs.

La répartition macroscopique des particules métalliques dans le support constitue également un critère important, principalement dans le cadre de réactions rapides et consécutives telles que les hydrogénations sélectives. Il faut généralement que ces éléments se situent dans une croûte à la périphérie du support afin d'éviter les problèmes de transfert de matière intragranulaire pouvant conduire à des défauts d'activité et une perte de sélectivité.

Ainsi, le document FR 2 922 784 décrit un catalyseur supporté comportant une croûte formée par des particules de palladium avec une répartition en taille de particules de palladium homogène et comprise entre 2 et 6 nm. Ces catalyseurs présentent des dispersions du palladium comprises entre 25 et 70% et des densités de particules de palladium comprises entre 1500 et 4100 particules palladium par $\mu m^2$ (noté Pd/$\mu m^2$). Ils comprennent en outre un composé alcalin ou alcalino-terreux réparti de façon homogène au sein du grain de support. Ce brevet décrit également une méthode de préparation de ce catalyseur par une suspension colloïdale de palladium.

Dans le cadre de l'hydrogénation de coupes légères, l'ajout d'au moins un second métal, de préférence un métal du groupe IB de préférence choisi par Ag, Au, Cu, de manière très préférence Ag, permet d'améliorer la sélectivité.

Ces effets bimétalliques sont en général liés à l'interaction créée entre les deux éléments. Il apparaît ainsi que l'identification d'un système catalytique multimétallique est conditionnée par l'établissement de cette interaction.

En effet, il est connu de l'homme de l'art que pour les réactions d'hydrogénation de molécules polyinsaturées telles que les dioléfines ou les acétyléniques, la vitesse de réaction dépend de la taille des particules métalliques, ce résultat est généralement décrit sous le terme « sensibilité à la structure ». Un maximum est généralement observé pour une taille de l'ordre de 3 à 4 nm, cette valeur pouvant varier en fonction notamment de la masse moléculaire des réactifs (décrit dans les documents M. Boudart, W.C. Cheng, J. Catal. 106, 1987, 134, et S. Hub, L. Hilaire, R. Touroude, Appl. Catal. 36, 1992, 307). Il est ainsi important d'obtenir une répartition en taille des particules centrée sur la valeur optimale ainsi qu'une dispersion minimale autour de cette valeur.

L'identification des couples bimétalliques a fait l'objet de très nombreux travaux dans le domaine des réactions d'hydrogénations sélectives (dans l'ouvrage V. Ponec, G.C. Bond, Catalysis by Metal and Alloys, Elsevier, Amsterdam, 1995). Ces études ont en outre révélé la difficulté à obtenir l'effet de synergie souhaitée, cet effet étant conditionné au mode de synthèse choisi. Ainsi, la composition locale de la phase active joue un rôle clé dans l'atteinte de performances catalytiques élevées. Les rendements obtenus étant la résultante des transformations se produisant sur chaque particule, la composition de celles-ci devra être ajustée à la formulation optimale et les particules devront être homogènes entre elles.

Enfin, la répartition macroscopique des éléments dans les billes de support constitue également un critère important. Il est préférable de déposer les éléments dans une fine croûte à la périphérie des grains de support.

Ainsi, le document FR 2 882 531 décrit un catalyseur bimétallique pour lequel la taille, la composition ainsi que la répartition des particules bimétalliques dans les billes de support sont adaptées aux exigences des réactions d'hydrogénation sélective. Les caractéristiques du catalyseur sont notamment obtenues par un procédé de préparation particulier comprenant non pas une méthode d'imprégnation classique mais l'utilisation de suspension colloïdale pour l'imprégnation. Plus particulièrement, le procédé de préparation comprend la préparation d'une suspension colloïdale d'oxyde d'un premier métal M1, la mise en contact d'un métal M2 dans une deuxième étape, la mise en contact avec le support dans une troisième étape, un séchage puis une éventuelle calcination.

Le document US2010/217052 décrit un catalyseur PdAg supporté, dans lequel le palladium et l'argent forment en partie un alliage dans une fine croûte périphérique, ce qui permet d'obtenir des catalyseurs particulièrement adaptés pour l'hydrogénation sélective de coupes C2. La proportion de particules formant un alliage est mesurée par adsorption de CO sur la surface catalytique. Le support utilisé est de très faible surface (1 à 80 m$^2$/g). Le procédé de préparation comprend une étape d'imprégnation des deux métaux dans une solution contenant de l'eau et un solvant organique miscible dans l'eau, un éventuel séchage et une calcination à une température maximale de 400°C. Le pourcentage deau dans la solution d'imprégnation permet de faire varier l'épaisseur de la croûte de palladium et d'argent.

Le document EP0780155 décrit un procédé de préparation d'un catalyseur d'hydrogénation sélective comprenant du palladium, de l'argent et un alcalin dans les proportions revendiquées sur un support d'alumine, ledit palladium et ledit argent étant répartis dans une croûte à la périphérie du support, et dans lequel au. moins 30 % des particules métalliques contiennent à la fois du palladium et de l'argent. Ce catalyseur est préparé par imprégnation du palladium et de l'argent soit par une solution commune soit à partir de solutions séparées. Dans ce dernier cas, une calcination peut être réalisée entre deux étapes d'imprégnation.

La présente invention vise à obtenir des catalyseurs ayant des très bonnes performances pour les procédés d'hydrogénation sélective des composés hydrocarbonés insaturés présents dans les coupes d'hydrocarbures issues du vapocraquage et/ou du craquage catalytique et en particulier dans les coupes C3.

La demanderesse a découvert, et ceci constitue l'objet de la présente invention, que les performances d'un catalyseur bimétallique PdAg en croûte pouvaient être nettement améliorées lorsque les atomes d'argent sont en étroite proximité des atomes de palladium dans la croûte contenant les deux métaux. En effet, la fine croûte du catalyseur comprend ainsi du palladium et de l'argent disposés d'une manière spécifique : la teneur locale en palladium à chaque point le long du diamètre du grain a la même évolution que la teneur locale en argent. En d'autres termes, lorsque la teneur locale en palladium à un point donné le long du diamètre augmente par rapport à un autre point donné le long du diamètre, la teneur en argent augmente également. D'une manière générale, on observe que la plus forte teneur en palladium et d'argent se trouvent proche de la surface du grain de support et diminue progressivement vers l'intérieure du grain. Cette évolution des teneurs en palladium et en argent peut s'exprimer par un Rapport de Proximité dit RP qui sera défini par la suite.

La proximité étroite, liée avec d'autres caractéristiques, telles que la surface spécifique du support, permet d'obtenir des catalyseurs particulièrement adaptés aux réactions d'hydrogénation sélective.

Cette proximité particulière du palladium et de l'argent sur le support est due au procédé de préparation tel que défini dans les revendications. En effet, le procédé de préparation comprend deux étapes bien distinctes, une première étape, dans laquelle le palladium est déposé par une méthode colloïdale, suivie d'une calcination, puis une deuxième étape, dans laquelle l'argent est déposé sur le catalyseur contenant le palladium ayant subi au préalable une réduction en phase liquide. La technique de suspension colloïdale permet d'obtenir des croûtes très fines avec des tailles de particules métalliques homogènes. De même, le procédé de préparation permet d'utiliser des supports ayant des surfaces spécifiques assez élevées, notamment de préférence entre 65 et 150 $m^2/g$. Ceci permet de mieux fixer les particules métalliques sur le support et de minimiser le frittage lors de la calcination effectuée à haute température (supérieure à 450°C). Un traitement thermique entre 450°C et 70°C permet d'améliorer le rapport de proximité RP entre les atomes du palladium et ceux de l'argent, ce qui permet d'obtenir des catalyseurs plus performants en hydrogénation sélective.

## DESCRIPTION DÉTAILLÉE DE L'INVENTION

### Caractéristique du catalyseur

Le procédé de préparation selon l'invention permet de préparer un catalyseur comprenant un grain de support poreux sur lequel est déposé du palladium et de l'argent et au moins un métal sélectionné dans le groupe constitué par les alcalins et les alcalino-terreux, le support poreux comprenant au moins un oxyde réfractaire sélectionné dans le groupe constitué par la silice, l'alumine et la silice-alumine, la surface spécifique du support poreux étant comprise entre 10 et 150 $m^2/g$, la teneur en palladium dans le catalyseur étant comprise entre 0,05 et 0,6 % poids, la teneur en argent dans le catalyseur étant comprise entre 0,02 et 3 % poids, au moins 80 % poids de palladium étant réparti dans une croûte à la périphérie du support, l'épaisseur de ladite croûte étant comprise entre 10 et 160 $\mu m$, au moins 80 % poids de l'argent étant réparti dans une croûte à la périphérie du support, l'épaisseur de ladite croûte étant comprise entre 10 et 160 $\mu m$, la teneur locale en palladium à chaque point le long du diamètre du grain ayant la même évolution que la teneur locale en argent, la somme des teneurs en métaux alcalins et/ou alcalino-terreux étant comprise entre 0,02 et 5 % poids.

De préférence, la surface spécifique dudit support poreux est comprise entre 65 et 150 $m^2/g$, dans lequel la teneur en palladium dans le catalyseur est comprise entre 0,05 et 0,4 % poids, la teneur en argent dans le catalyseur est comprise entre 0,05 et 0,3 % poids, au moins 80 % poids de palladium est réparti dans une croûte à la périphérie du support, l'épaisseur de ladite croûte est comprise entre 10 et 110 $\mu m$, au moins 80 % poids de l'argent est réparti dans une croûte à la périphérie du support, l'épaisseur de ladite croûte est comprise entre 10 et 110 $\mu m$.

Selon l'invention, le grain de support poreux se présente avantageusement sous forme de billes, de trilobes, d'extrudés, de pastilles, ou d'agglomérats irréguliers et non sphériques dont la forme spécifique peut résulter d'une étape de concassage. De manière très avantageuse, ledit support se présente sous forme de billes ou d'extrudés. De manière encore plus avantageuse, ledit support se présente sous forme de billes.

Le volume poreux du support est généralement compris entre 0,1 et 1,5 cm$^3$/g, de préférence compris entre 0,3 et 1,3 cm$^3$/g.

Le support poreux comprend au moins un oxyde réfractaire sélectionné dans le groupe constitué par la silice, l'alumine et la silice-alumine. De préférence, le support est de l'alumine.

Le métal alcalin est généralement sélectionné dans le groupe constitué par le lithium, le sodium, le potassium, le rubidium et le césium, de préférence par le lithium, le sodium et le potassium, de manière très préférée par le sodium et le potassium. De manière encore plus préférée, le métal alcalin est le sodium.

Le métal alcalino-terreux est généralement sélectionné dans le groupe constitué par le magnésium, le calcium, le strontium et le baryum, de préférence par le magnésium et le calcium, de manière très préférée par le magnésium.

Le métal alcalin, lorsqu'il est présent, est de préférence réparti de manière homogène à travers le support avec un coefficient R, défini ci-après, compris entre 0,8 et 1,2.

Le métal alcalino-terreux, lorsqu'il est présent, est de préférence réparti de manière homogène à travers le support avec un coefficient R, défini ci-après, compris entre 0,8 et 1,2.

Le catalyseur préparé par le procédé de la présente invention peut être caractérisé par plusieurs paramètres qui seront décrits par la suite, notamment :

- le coefficient R qui exprime la répartition homogène de l'élément alcalin et/ou alcalino-terreux à travers le grain de support,
- l'épaisseur de la croûte,
- le rapport de proximité RP qui exprime la proximité des atomes de palladium avec les atomes d'argent à un point donné dans la croûte,
- la dispersion métallique qui permet de déduire la taille moyenne des particules métalliques.

Coefficient R

Les profils de répartition des éléments au sein des grains de catalyseurs sont obtenus par microsonde de Castaing. Au moins 30 points d'analyses sont effectués le long du diamètre de la bille ou de l'extrudé à raison d'une dizaine de points sur la croûte de l'élément actif et d'une dizaine de points au centre du grain. Il est ainsi obtenu le profil de répartition $c(x)$ pour $x \in [-r;+r]$ avec c la concentration locale de l'élément, r le rayon de la bille ou de l'extrudé et x la position du point d'analyse le long du diamètre du grain par rapport au centre de ce grain.

La répartition des éléments est caractérisée par un coefficient R adimensionnel pondérant la concentration locale par un poids croissant en fonction de la position sur le diamètre. Par définition :

$$R = \int_{-r}^{r} c(x)x^2 dx \bigg/ \frac{r^2}{3} \int_{-r}^{r} c(x) dx$$

Ainsi un élément dont la concentration est uniforme présente un coefficient R égal à 1, un élément déposé en dôme (concentration au coeur plus importante que la concentration aux bords du support) présente un coefficient supérieur à 1 et un élément réparti en croûte (concentration aux bords plus importante que la concentration au coeur du support) présente un coefficient inférieur à 1. L'analyse par microsonde de Castaing donne les valeurs des concentrations en un nombre fini de valeurs de x, R est ainsi évalué numériquement par des méthodes d'intégration bien connues de l'homme du métier. De préférence, R est déterminé par la méthode des trapèzes.

La répartition de l'élément alcalin est définie comme homogène lorsque le coefficient de répartition R défini ci-dessus est compris entre 0,8 et 1,2.

La répartition de l'élément alcalino-terreux est définie comme homogène lorsque le coefficient de répartition R défini ci-dessus est compris entre 0,8 et 1,2.

Épaisseur de croûte

Lorsque le palladium et l'argent sont repartis en croûte, leur concentration locale diminue généralement progressivement lorsqu'elle est mesurée en partant du bord du grain catalytique vers l'intérieur. Une distance du bord du grain, à laquelle la teneur locale en palladium et en argent devient nulle, peut souvent ne pas être déterminée avec précision et reproductibilité. Afin de mesurer une épaisseur de croûte qui soit significative pour la majorité des particules de palladium et d'argent, l'épaisseur de croûte est définie comme la distance au bord du grain contenant 80 % de l'élément.

A partir du profil de répartition obtenu par microsonde de Castaing, on calcule la quantité cumulée $Q(y)$ de l'élément dans le grain en fonction de la distance y au bord du grain.

Pour une bille :

$$Q(y) = \int_{-r}^{-y} c(x)4\pi . x^2 dx + \int_{y}^{r} c(x)4\pi . x^2 dx$$

Pour un extrudé :

$$Q(y) = \int_{-r}^{-y} c(x)2\pi . x dx + \int_{y}^{r} c(x)2\pi . x dx$$

$Q(r)$ correspond ainsi à la quantité totale de l'élément dans le grain. On résout ensuite numériquement l'équation suivante en y :

$$\frac{Q(y)}{Q(r)} = 0.8$$

c étant une fonction strictement positive, Q est donc une fonction strictement croissante et cette équation possède une seule solution qui est l'épaisseur de croûte.

Rapport de proximité RP

Les catalyseurs PdAg sont caractérisés par microsonde de Castaing. Cette analyse permet de connaitre localement la concentration massique en métal Pd, Ag.

Pour un catalyseur, cette analyse permet de déterminer la répartition relative des deux métaux le long du grain catalytique par intégration d'une succession d'analyses FX à une distance y au bord du grain. La formule permettant d'estimer la proximité des deux métaux est la suivante:

$$\text{Rapport de proximité} = \quad RP(y) = \frac{Q(y)Pd \, / \, Q(r)Pd}{Q(y)Ag \, / \, Q(r)Ag}$$

avec :

Q (y) Pd = Somme des concentrations en palladium entre le bord du grain et une distance y du bord du grain catalytique (%pds)

Q (y) Ag = Somme des concentrations en argent entre le bord du grain et une distance y du bord du grain catalytique (%pds)

Q (r) Pd = teneur en palladium totale du grain catalytique (%pds)

Q (r) Ag= teneur en argent totale du grain catalytique (%pds).

On définit ainsi un critère de proximité, qui rend compte de la localisation relative des deux métaux dans le support. Ce dernier, déterminé par microsonde représente le rapport massique en tout point y du support des éléments métalliques ajoutés, dans notre cas Pd et Ag. Le rapport de proximité d'un catalyseur contant des métaux localement uniformément répartis sera de 1.

Dans le catalyseur selon l'invention, le rapport de proximité RP est compris entre 0,5 et 2, de préférence entre 0,8 et 1,4.

Dispersion métallique D

Les mesures de dispersion métallique sont effectuées pour déduire la taille moyenne des particules de palladium. Ces mesures sont effectuées après la première étape du procédé, dans laquelle uniquement le palladium est introduit sur le support.

Les mesures de dispersions métalliques sont effectuées par chimisorption de monoxyde de carbone CO, sur le catalyseur préalablement réduit sous 1 litre d'hydrogène par heure et par gramme de catalyseur, avec une montée en température de 300°C/h et un palier de deux heures à 150°C. Le catalyseur est ensuite balayé pendant 1 heure à 150°C sous hélium puis refroidi jusqu'à 25°C sous hélium.

La chimisorption de CO est réalisée à 25°C en dynamique selon les pratiques habituelles connues de l'homme du métier, elle conduit à un volume de CO chimisorbé, à partir duquel l'homme du métier peut calculer le nombre de molécules de CO chimisorbées.

Un rapport stoechiométrique d'une molécule de CO par atome de Pd de surface est pris en compte pour calculer le nombre d'atomes de Pd de surface. La dispersion est exprimée en % d'atomes de Pd de surface par rapport à la totalité des atomes de Pd présent dans l'échantillon du catalyseur.

La dispersion métallique D du palladium est généralement comprise entre 10% et 70%, de préférence entre 15 et 60%.

La taille moyenne des cristallites est déduite de ces mesures de dispersion, en appliquant les relations dispersion-taille de particules connues de l'homme du métier et décrites dans le document "Analyse physico-chimique des catalyseurs industriels, Chapitre I, Éditions Technip, Paris 2001".

La répartition en taille de particules du catalyseur de palladium est homogène, elle est comprise entre 2 et 6 nm.

Après introduction de l'argent dans le catalyseur (deuxième étape), la dispersion métallique du palladium baisse. On parlera ainsi de la dispersion "apparente", elle est comprise entre 5 et 50%. L'introduction de l'argent ne fait par contre pas changer la répartition en taille des particules du catalyseur qui restera comprise entre 2 et 6 nm.

Densité de particules de palladium De

On peut également caractériser le catalyseur par la densité de particules de palladium, dénommée De, telle que défini dans FR2922784.

Dans le cas d'une bille de support de volume Vt et de rayon r dans laquelle le palladium est présent sous la forme d'une croûte d'épaisseur e, la densité de particules de palladium, est obtenue par la formule suivante:

$$De = \frac{(\%Pd).\mathrm{N}_a}{S_{BET}.M_{Pd}.n_{Pd}.10^{14}} \frac{V_t}{V_c} \quad (1)$$

De : Densité de particules (en nombre de particules de palladium par $\mu m^2$) %Pd : Teneur massique en Pd (gramme de Pd pour 100 grammes de catalyseur)

$N_a$ : Nombre d'Avogadro ($6,022.10^{23}$ atomes/mole)

$S_{BET}$ : Surface spécifique du support (en $m^2/g$)

$M_{Pd}$ : Masse molaire du palladium (106,42 g/mole)

$n_{Pd}$ : nombre d'atomes de palladium par particule de palladium.

$V_t$ : Volume total de la bille de support en $mm^3$

$V_t = 4/3.\pi.r^3$ (r étant le rayon de la bille)

$V_c$ : Volume de la croûte en $mm^3$

$V_c = V_t - 4/3.\pi.(r-e)^3$ (e étant l'épaisseur de la croûte)

Le nombre d'atomes de Pd par particule métallique en fonction de la taille de la particule est déterminé par le modèle de Van Hardeveld et Hartog (décrit dans le document R. Van Hardeveld, F. Hartog, Surf. Sci., 15 (1969) 189).

L'homme du métier peut calculer la densité de particules de palladium par une formule mathématique dépendant de la forme du support considéré. Ainsi, pour un support dont la forme est différente d'une bille, l'équation de la densité est toujours valable, mais les formules pour calculer $V_t$ et $V_c$ doivent être adaptées par l'homme du métier en fonction de la géométrie du support.

La densité de particules de palladium dans la croûte notée De est avantageusement comprise entre 1500 et 4100 particules de palladium par $\mu m^2$, de préférence comprise entre 1550 et 4000 particules de palladium par $\mu m^2$, de préférence comprise entre 1600 et 3950 particules de palladium par $\mu m^2$.

## Procédé de préparation du catalyseur

L'invention concerne un procédé de préparation du catalyseur selon la revendication 1 comprenant d'une manière générale les étapes suivantes:

- une première étape, dite étape 1, dans laquelle le palladium est déposé par une méthode colloïdale, suivie d'un séchage et d'une calcination,
- une deuxième étape, dite étape 2, dans laquelle l'argent est déposé, après une réduction en phase liquide du catalyseur contenant le palladium, suivie d'un séchage et d'une calcination.

Il est important de souligner que le procédé de préparation est effectué en deux étapes distinctes incluant une étape de calcination intermédiaire entre les deux étapes de dépôts de métal. Ainsi, on introduit d'abord le palladium par voie colloïdale, puis on sèche et on calcine, et on introduit par la suite l'argent, précédé d'une étape de réduction du catalyseur en phase liquide, puis on sèche et on calcine de préférence à haute température.

Plus particulièrement, le procédé de préparation du catalyseur comprend :

- une étape dans laquelle on introduit le palladium sur le support, dite étape 1, comprenant les étapes suivantes :

  - une étape 1a), dans laquelle on prépare dans un appareillage une suspension colloïdale d'oxyde de palladium ou d'hydroxyde de palladium en phase aqueuse par mélange d'une solution aqueuse 1 comprenant au moins un hydroxyde sélectionné dans le groupe constitué par les hydroxydes d'alcalins et les hydroxydes d'alcalino-terreux et d'une solution aqueuse 2 comprenant au moins un précurseur du palladium, la solution 2 puis la solution 1 étant versées dans l'appareillage ou les solutions 1 et 2 étant versées simultanément dans l'appareillage,
  - une étape 1b), dans laquelle on imprègne ladite suspension colloïdale sur un grain de support poreux ayant une surface spécifique comprise entre 10 et 150 m$^2$/g,
  - une étape 1c), dans laquelle on mature ledit support imprégné obtenu à l'étape 1b),
  - une étape 1d), dans laquelle on sèche le catalyseur obtenu à l'étape 1c),
  - une étape 1e), dans laquelle on calcine le catalyseur obtenu à l'étape 1d),

- puis une étape dans laquelle on introduit l'argent, dite étape 2, comprenant les étapes suivantes :

  - une étape 2a), dans laquelle on réduit le catalyseur préparé selon l'étape 1 par mise en contact avec une solution aqueuse comprenant au moins un agent réducteur en phase liquide,
  - une étape 2b), dans laquelle on filtre le catalyseur obtenu à l'étape 2a),
  - une étape 2c), dans laquelle on imprègne le catalyseur préparé selon l'étape 2b) par mise en contact sous agitation avec une solution aqueuse comprenant un sel précurseur de l'argent,
  - une étape 2d), dans laquelle on filtre le catalyseur obtenu à l'étape 2c),
  - une étape 2e), dans laquelle on sèche le catalyseur obtenu à l'étape 2d),
  - une étape 2f), dans laquelle on calcine le catalyseur obtenu à l'étape 2e), de préférence entre 450°C et 700°C.

Les différentes étapes 1 et 2 sont par la suite détaillées.

## Étape 1 : Dépôt du palladium par une méthode colloïdale

Étape 1a) Préparation d'une suspension colloïdale de palladium

La suspension colloïdale est généralement obtenue par hydrolyse du cation palladium en milieu aqueux, ce qui conduit à la formation de particules d'oxydes ou d'hydroxydes de palladium en suspension.

La solution aqueuse d'hydroxydes d'alcalins ou d'hydroxydes alcalino-terreux est généralement sélectionnée dans le groupe constitué par les solutions aqueuses de soude et les solutions aqueuses d'hydroxyde de magnésium. De manière préférée on utilise une solution d'hydroxyde de potassium, de manière encore plus préférée, une solution aqueuse de soude.

Le précurseur du palladium est généralement sélectionné dans le groupe constitué par le chlorure de palladium, le nitrate de palladium et le sulfate de palladium. De manière très préférée, le sel précurseur du palladium est le nitrate de palladium.

La solution aqueuse 2 comprenant au moins un sel précurseur du palladium puis la solution aqueuse 1 comprenant au moins un hydroxyde d'alcalins ou d'alcalino-terreux sont généralement versées dans l'appareillage. Les solutions 1 et 2 peuvent être versées simultanément dans l'appareillage. De préférence, la solution aqueuse 2 puis la solution aqueuse 1 est versée dans l'appareillage.

La suspension colloïdale reste généralement dans l'appareillage pendant un temps de séjour compris entre 0 et 20 h. Le pH de la suspension colloïdale peut être modifié pendant ce temps de séjour par ajout de quantités d'acide ou de base compatibles avec la stabilité de la suspension colloïdale.

En général, la température de préparation est comprise entre 5°C et 40°C et de manière préférée entre 15°C et 35°C. La concentration en palladium est de préférence comprise entre 2 et 100 mmoles par litres, de manière plus préférée entre 4 et 50 mmoles par litre.

Les concentrations des solutions 1 et 2 sont généralement choisies afin d'obtenir un pH de la suspension colloïdale compris entre pH = 1,0 et pH = 3,5.

Étape 1b) : Dépôt de la suspension colloïdale par imprégnation sur un support,

La suspension colloïdale préparée à l'étape 1a) est ensuite imprégnée sur un support.

Le support peut éventuellement subir un ensemble de traitements avant l'étape d'imprégnation, tels que des calcinations ou des hydratations. Le support peut aussi déjà comprendre un ou plusieurs éléments métalliques avant l'imprégnation de la suspension colloïdale. Des éléments métalliques peuvent aussi être introduits dans la suspension colloïdale. Ces éléments métalliques peuvent être introduits soit par des techniques conventionnelles, soit en utilisant le procédé selon la présente invention.

La suspension colloïdale est de préférence versée sur le support. Le volume de la suspension colloïdale est généralement compris entre 0,9 et 1,1 fois le volume poreux du support.

Ce processus peut être réalisé soit de façon discontinue, c'est-à-dire que l'étape de préparation de la suspension colloïdale précède l'étape d'imprégnation sur le support et que l'essentiel de la suspension colloïdale est envoyée en une seule fois vers l'étape d'imprégnation, soit en continu, c'est-à-dire que le produit obtenu dans l'étape 1a) est envoyé en continu après ajustement du temps de séjour de la suspension colloïdale à l'étape 1b).

On peut par exemple citer comme processus continu, un processus où les solutions 1 et 2 sont versées simultanément dans un bac qui se déverse en continu dans une zone comprenant le support à imprégner.

Étape 1c) Maturation

Après imprégnation, le support imprégné est généralement maturé à l'état humide, de préférence pendant 0,5 à 40 h, de manière très préférée pendant 1 à 30 h.

Étape 1d) Séchage

Le précurseur du catalyseur est généralement séché afin d'éliminer toute ou une partie de l'eau introduite lors de l'imprégnation, de préférence à une température comprise entre 50°C et 250°C, de manière plus préférée entre 70°C et 200°C. La durée du séchage est généralement comprise entre 0,5 h et 20 h.

Le séchage est généralement effectué sous air, ou sous air de combustion d'un hydrocarbure, de préférence du méthane. Les flux d'air cités peuvent contenir entre 0 et 80 grammes d'eau par kilogramme d'air, un taux d'oxygène compris entre 5% et 25% volume et un taux de dioxyde de carbone compris entre 0% et 10% volume.

Étape 1e) Calcination

Après séchage, le catalyseur est calciné, généralement sous air, ou sous air de combustion d'un hydrocarbure, de préférence du méthane. Les flux d'air cités peuvent contenir entre 0 et 80 grammes d'eau par kilogramme d'air, un taux d'oxygène compris entre 5% et 25% volume et un taux de dioxyde de carbone compris entre 0% et 10% volume. La température de calcination est généralement comprise entre 250°C et 900°C, de préférence comprise entre environ 300°C et environ 500°C. La durée de calcination est généralement comprise entre 0,5 h et 5 h.

Selon une première variante de cette étape 1 du procédé, le pH, le temps de séjour de la suspension colloïdale et la surface spécifique du support sont fixés dans leurs intervalles respectifs. La teneur en palladium comprise entre 0,05 et 0,6 % poids est alors ajustée afin d'obtenir une densité de particules de palladium dans la croûte comprise entre 1500 et 4100 particules de palladium par $\mu m^2$.

Selon une deuxième variante de cette étape 1 du procédé, la teneur en palladium comprise entre 0,05 et 0,6 % poids, le temps de séjour de la suspension colloïdale, la surface spécifique du support sont fixés dans leurs intervalles respectifs. Le pH est alors ajusté afin d'obtenir une densité de particules de palladium dans la croûte comprise entre 1500 et 4100 particules de palladium par $\mu m^2$.

On obtient après cette étape 1 un précurseur du catalyseur supporté comportant une croûte formée par des particules de palladium avec une répartition en taille de particules de palladium homogène et comprise entre 2 et 6 nm, tel que décrit dans FR2922784. Ces catalyseurs présentent des dispersions du palladium comprises entre 25 et 70% et des densités de particules de palladium comprises entre 1500 et 4100 particules Palladium par $\mu m^2$ (noté Pd/$\mu m^2$). Ils comprennent en outre un composé alcalin ou alcalino-terreux réparti de façon homogène au sein du grain de support.

**Étape 2 : Dépôt d'argent après réduction du catalyseur**

Étape 2a) Réduction du catalyseur préparé selon l'étape 1

Le catalyseur préparé selon l'étape 1 est ensuite réduit en phase liquide. Le volume de la solution aqueuse de réduction du catalyseur préparé selon l'étape 1 est généralement compris entre 1 et 20 fois le volume de catalyseur engagé.

L'agent réducteur utilisé est choisi parmi l'acide formique, l'acide citrique, l'acide ascorbique, l'acide oxalique, le formiate de sodium, l'acétate de sodium, le borohydrure de sodium, le formaldéhyde, le dextrose, l'hydrazine, l'hydrogène ou tout autre réducteur en phase liquide conventionnel. Le rapport molaire agent réducteur / palladium est de préférence compris entre 1 et 20, de préférence 2 et 15.

La température de préparation est de préférence comprise entre 5°C et 70°C, et de manière préférée entre 10°C et 60°C.

Le temps de séjour de ladite solution aqueuse dans l'appareillage étant compris entre 0 et 20 h.

De manière préférée, l'atmosphère est contrôlée, de préférence avec un gaz inerte ou réducteur. De manière très préférée un gaz inerte est utilisé comme atmosphère de réaction.

Étape 2b) Filtration

Le catalyseur réduit à l'étape 2a) est filtré partiellement ou totalement selon toutes les techniques connues de l'Homme du métier. Cette étape vise à enlever une partie ou la totalité des eaux de réduction.

Optionnellement, l'étape 2b) peut comprendre une ou plusieurs étapes de lavage(s) du solide, de préférence avec de l'eau, suivies de l'étape de filtration. Le volume total d'eau engagé pour le (s) étape(s) de lavage est compris entre 1 et 30 fois le volume catalytique engagé.

Étape 2c) Dépôt d'argent

Le catalyseur réduit, optionnellement lavé, et filtré, est mis en contact, sous agitation, avec une solution aqueuse comprenant le précurseur d'argent afin d'introduire l'argent dans le catalyseur.

Le précurseur d'argent est sélectionné dans le groupe constitué par le nitrate d'argent, l'acétate d'argent, le citrate d'argent, le chlorure d'argent, l'oxalate d'argent ou tout autre précurseur d'argent conventionnel.

Le volume de la solution aqueuse de précurseur d'argent est de préférence compris entre 1 et 20 fois le volume catalytique engagé. Le rapport molaire argent/ palladium est généralement compris entre 0,1 et 10, de préférence 0,1 et 5 de manière très préférée 0,2 et 2.

En général, la température de la solution est maintenue constante et est comprise entre 5°C et 70°C et de manière préférée entre 10°C et 60°C. Le temps de séjour de ladite solution aqueuse dans l'appareillage est de préférence compris entre 0,5 et 20 heures.

L'atmosphère de réaction peut être contrôlée, de préférence avec un gaz inerte ou réducteur. De manière très préférée, si l'atmosphère est contrôlée un gaz inerte est utilisé comme atmosphère de réaction.

Étape 2d) Filtration

Le catalyseur préparé selon l'étape 2c) est filtré partiellement ou totalement selon toutes les techniques connues de l'Homme du métier.

Optionnellement, l'étape 2d) peut comprendre une ou plusieurs étapes de lavage(s) du solide, de préférence avec de l'eau, suivies de l'étape de filtration. Le volume total d'eau engagé pour le (s) étape(s) de lavage est compris entre 1 et 30 fois le volume catalytique engagé.

Étape 2e) Séchage

Le catalyseur est généralement séché afin d'éliminer toute ou une partie de l'eau introduite lors de l'imprégnation, de préférence à une température comprise entre 50 et 250°C, de manière plus préférée entre 70°C et 200°C. La durée du séchage est de préférence comprise entre 0,5h et 20h.

Le séchage est généralement effectué sous air, ou sous air de combustion d'un hydrocarbure, de préférence du méthane. Les flux d'air cités peuvent contenir entre 0 et 80 grammes d'eau par kilogramme d'air, un taux d'oxygène compris entre 5% et 25% volume et un taux de dioxyde de carbone compris entre 0% et 10%.

Étape 2f) Calcination

Après séchage le catalyseur est calciné, généralement sous air, ou sous air de combustion d'un hydrocarbure, de préférence du méthane. Les flux d'air cités peuvent contenir entre 0 et 80 grammes d'eau par kilogramme d'air, un taux d'oxygène compris entre 5% et 25% volume et un taux de dioxyde de carbone compris entre 0% et 10% volume.

La température de calcination est généralement comprise entre 450°C et 900°C, de préférence comprise entre 450°C et 700°CLa durée de calcination est généralement comprise entre 0,5 heures et 5 heures.

Avant utilisation, le catalyseur est généralement activé par un traitement sous flux d'hydrogène à une température comprise entre la température ambiante et environ 500°C, préférentiellement entre 80 et 180°C, de manière encore plus préférée entre 100°C et 160°C. Ce traitement peut être effectué dans le réacteur même où se réalisera la réaction catalytique (réduction in situ) ou de manière préalable dans un équipement indépendant (réduction hors site ou ex situ).

La réduction est réalisée en présence d'un gaz réducteur comprenant entre 25 vol% et 100 vol% d'hydrogène, de préférence 100% volume d'hydrogène. L'hydrogène est éventuellement complété par un gaz inerte pour la réduction, de préférence de l'argon, de l'azote ou du méthane.

La réduction comprend généralement une phase de montée en température puis un palier.

La durée du palier de réduction est généralement comprise entre 1 et 10 heures, de préférence entre 2 et 8 heures.

La VVH est généralement comprise entre 150 et 3000, de préférence entre 300 et 1500 litres de gaz réducteur par heure et par litre de catalyseur. Le catalyseur susceptible d'être obtenu à partir du procédé de préparation de catalyseur selon la présente invention est aussi décrit.

**Utilisation du catalyseur selon l'invention**

Le catalyseur préparé par le procédé selon l'invention peut être utilisé dans les réactions d'hydrogénation des composés comportant des fonctions acétyléniques, diéniques, oléfiniques. Il est possible de mettre en oeuvre un procédé d'hydrogénation sélective par mise en contact d'une charge sur le catalyseur selon l'invention ou sur le catalyseur préparé selon l'invention, ladite charge étant sélectionnée dans le groupe constitué par les coupes C3, les coupes C4, les coupes C5 de vapocraquage et/ou de craquage catalytique et les essences de vapocraquage appelée aussi essences de pyrolyse, de manière préférée les charges sont des coupes C3 de vapocraquage et/ou de craquage catalytique.

Selon une application préférée, le catalyseur préparé par le procédé selon l'invention est mis en oeuvre pour les réactions d'hydrogénation sélective des coupes polyinsaturées d'hydrocarbures issues de vapocraquage et/ou de craquage catalytique, de préférence des coupes polyinsaturées d'hydrocarbures issues du vapocraquage.

Les procédés de conversion des hydrocarbures tels que le vapocraquage ou le craquage catalytique sont opérés à haute température et produisent une grande variété de molécules insaturées telles que l'éthylène, le propylène, les butènes linéaires, l'isobutène, les pentènes ainsi que des molécules insaturés contenant jusqu'à environ 15 atomes de carbone.

En parallèle sont également formés des composés polyinsaturés: acétylène, propadiène et méthylacétylène (ou propyne), 1-2 et 1-3 butadiène, vinylacétylène et éthylacétylène, et d'autres composés polyinsaturés dont le point d'ébullition correspond à la fraction essence C5+.

Tous ces composés polyinsaturés doivent être éliminés pour permettre l'utilisation de ces différentes coupes dans les procédés de pétrochimie tels que les unités de polymérisation.

Le procédé d'hydrogénation sélective s'est progressivement imposé pour éliminer les composés polyinsaturés des coupes pétrolières C3 à C5 et de l'essence de pyrolyse car ce procédé permet la conversion des composés les plus insaturés vers les alcènes correspondants en évitant la saturation totale et donc la formation des alcanes correspondant.

Ainsi, par exemple, la coupe C3 de vapocraquage peut avoir la composition moyenne suivante : de l'ordre de 90% poids propylène, de l'ordre de 3 à 8% poids de propadiène et méthylacétylène, le reste étant essentiellement du propane. Dans certaines coupes C3, entre 0,1 et 2% poids de C2 et de C4 peut aussi être présent. Les spécifications concernant les concentrations de ces composés polyinsaturés pour les unités de pétrochimie et de polymérisation sont très basses : 20-30 ppm poids de MAPD (méthylacétylène et propadiène) pour le propylène qualité chimique et moins de 10 ppm poids voire jusqu'à 1 ppm poids pour la qualité « polymérisation ».

Une coupe C4 de vapocraquage présente par exemple la composition molaire moyenne suivante : 1 % de butane, 46,5% de butène, 51% de butadiène, 1,3% de vinylacetylène (VAC) et 0,2% de butyne. Dans certaines coupes C4, entre 0,1 et 2% poids de C3 et de C5 peut aussi être présent. La encore les spécifications sont sévères : teneur en dioléfines strictement inférieure à 10 ppm poids pour une coupe C4 qui sera utilisée en pétrochimie ou polymérisation.

Une coupe C5 de vapocraquage présente par exemple la composition moyenne en masse suivante : 21% de pentanes, 45% de pentènes, 34% de pentadiènes.

L'essence de pyrolyse correspond à une coupe dont la température d'ébullition est généralement comprise entre 0°C et 250°C, de préférence entre 10°C et 220°C. Cette charge comprend généralement la coupe C5-C12 avec des traces de C3, C4, C13, C14, C15 (par exemple entre 0,1 à 3% poids pour chacune de ces coupes). Par exemple, une coupe C5-200°C a généralement une composition en % poids suivante:

- Paraffines 8 - 12
- Aromatiques 58 - 62
- Mono-oléfines 8 - 10
- Dioléfines 18 - 22
- Soufre 20 - 300 ppm

L'hydrogénation sélective peut être réalisée en phase gaz ou liquide, de préférence en phase liquide. En effet, une réaction en phase liquide permet d'abaisser le coût énergétique et d'augmenter la durée de cycle des catalyseurs.

D'une manière générale, l'hydrogénation sélective s'effectue à une température est comprise entre 0°C et 500°C, une pression comprise entre 0,1 et 20 MPa, une vitesse volumique horaire comprise entre 0,1 et 50 $h^{-1}$ pour une charge liquide, entre 500 et 30 000 $h^{-1}$ pour une charge gazeuse.

Plus particulièrement, dans le cas d'une hydrogénation sélective d'une charge C3 à C5, et pour une réaction en phase liquide, la pression est généralement comprise entre 1 et 3 MPa, la température entre 2 et 200°C et le ratio molaire hydrogène/(composés polyinsaturés à hydrogéner) entre 0,1 et 10, de préférence entre 1 et 8. Les vitesses horaires volumiques sont comprises entre 1 et 200$h^{-1}$.

Dans le cas d'une hydrogénation sélective d'une charge C3 à C5, et pour une réaction d'hydrogénation en phase gazeuse, la pression est généralement comprise entre 1 et 3 MPa, la température entre 40 et 120°C et le ratio molaire hydrogène/(composés polyinsaturés à hydrogéner) entre 0,1 et 4, de préférence entre 1 et 2. Les vitesses horaires volumiques sont comprises entre 1 et 15000 $h^{-1}$.

Dans le cas d'une hydrogénation sélective d'essence de pyrolyse, le ratio molaire hydrogène/(composés polyinsaturés à hydrogéner) est généralement compris entre 1 et 2, la température est généralement comprise entre 40°C et 200°C, de préférence entre 50 et 180°C, la vitesse horaire spatiale (correspondant au volume d'hydrocarbure par volume de catalyseur et par heure) est comprise généralement entre 0,5 $h^{-1}$ et 10 $h^{-1}$, de préférence entre 1 $h^{-1}$ et 5 $h^{-1}$ et la pression est généralement comprise entre 1,0 MPa et 6,5 MPa, de préférence entre 2,0 MPa et 3,5 MPa.

Le débit d'hydrogène est ajusté afin d'en disposer en quantité suffisante pour hydrogéner théoriquement l'ensemble des dioléfines, des acétyléniques et des alkényl aromatiques et de maintenir un excès d'hydrogène en sortie de réacteur. Afin de limiter le gradient de température dans le réacteur, il peut être avantageux de recycler une fraction de l'effluent à l'entrée et/ou au milieu du réacteur.

## EXEMPLES

Les exemples suivants illustrent l'invention sans en limiter la portée :

**Exemple 1** : préparation d'un catalyseur C1 selon l'invention

Cet exemple montre la préparation d'un catalyseur selon l'invention comprenant l'imprégnation du support en deux étapes selon le procédé de l'invention, c'est-à-dire par utilisation d'une solution colloïdale pour le Pd. Le catalyseur obtenu comprend une fine croûte de palladium et d'argent présentant un rapport de proximité RP dans une gamme de 0,5 à 2.

Une suspension colloïdale d'oxyde de Pd est préparée sous agitation à 25°C par dilution de 1,8 g d'une solution de nitrate de palladium $Pd(NO_3)_2$ contenant 8,5 % poids de palladium Pd avec environ 45 ml d'eau déminéralisée, puis ajout d'environ 10 ml d'une solution de soude pour arriver à un pH de 2,4. La suspension est ensuite diluée avec de l'eau déminéralisée à un volume qui correspond au volume poreux du support alumine. Cette solution est ensuite imprégnée sur 80g d'une alumine dont la surface spécifique est de 71 $m^2$/g mise en forme sous forme de billes. Une étape de maturation du support imprégné avant séchage d'une durée de 20 h est effectuée sous air en milieu confiné et humide. Le solide obtenu est séché sous air pendant 2 h à 120°C. Le catalyseur est ensuite calciné sous unflux d'air 2 h à 450°C.

Le solide obtenu est immergé dans 500ml d'une solution 1,4g/l d'acide formique. La solution est agitée pendant 2h à 25°C.Le solide est ensuite filtré et lavé en trois fois avec 500ml d'eau. Le solide réduit est alors immergé dans 500ml d'une solution à 0,23 g/L de nitrate d'argent à 30°C pendant 5h. Le solide obtenu est filtré, lavé en trois fois avec 500ml d'eau puis séché 3h à 120°C et calciné 2h à 570°C.

Le catalyseur C1 ainsi préparé contient 0,19% poids de palladium et 0,10% poids d'argent.

La caractérisation du catalyseur C1 par microsonde de Castaing montre que 80% du Pd est réparti sur une croûte d'épaisseur environ 80 $\mu$m. 80% de l'argent est réparti dans croûte d'épaisseur environ 110 $\mu$m. Le sodium est réparti de façon homogène avec un coefficient de répartition R(Na)=0,92. La dispersion apparente du palladium du catalyseur C1 est de 17%. Le facteur de proximité RP est de 1,40.

**Exemple 2** : préparation d'un catalyseur C2 selon l'invention

Cet exemple est identique à l'exemple 1 à l'exception de la concentration du palladium qui est moitié moins élevée que dans l'exemple 1. Ceci conduit à un catalyseur avec une croûte plus fine et un meilleur facteur de proximité RP.

Une suspension colloïdale d'oxyde de Pd est préparée sous agitation à 25°C par dilution de 0,95 g d'une solution de nitrate de palladium Pd(NO$_3$)$_2$ contenant 8,5% poids de palladium Pd avec environ 45 ml d'eau déminéralisée, puis ajout d'environ 10 ml d'une solution de soude pour arriver à un pH de 2,4. La suspension est ensuite diluée avec de l'eau déminéralisée à un volume qui correspond au volume poreux du support alumine. Cette solution est ensuite imprégnée sur 80g d'une alumine dont la surface spécifique est de 71 m$^2$/g mise en forme sous forme de billes. Une étape de maturation du support imprégné avant séchage d'une durée de 20 h est effectuée sous air en milieu confiné et humide. Le solide obtenu est séché sous air 2 h à 120°C. Le catalyseur est ensuite calciné sous un flux d'air 2 h à 450°C.

Le solide obtenu est immergé dans 400ml d'une solution 0,87g/l d'acide formique. La solution est agitée pendant 2h à 25°C.Le solide est ensuite filtré et lavé en trois fois avec 500ml d'eau. Le solide réduit est immergé dans 500ml d'une solution à 0,23 g/l de nitrate d'argent à 30°C pendant 5h. Le solide obtenu est filtré, lavé en trois fois avec 500ml d'eau puis séché 2 h à 120°C et calciné 3 heures à 570°C.

Le catalyseur C2 contient 0,1% poids de palladium et 0,1% poids d'argent.

La caractérisation du catalyseur par microsonde de Castaing montre que 80% du palladium est réparti sur une croûte d'épaisseur environ 40 $\mu$m. 80% de l'argent est réparti sur une croûte d'épaisseur environ 70$\mu$m. Le Na est réparti de façon homogène avec un coefficient de répartition R(Na)=0,93. La dispersion apparente du palladium est de 16%. Le facteur de proximité RP est de 1,27.

**Exemple 3** : préparation d'un catalyseur C3 non conforme à l'invention

Cet exemple montre la préparation d'un catalyseur non conforme à l'invention comprenant l'imprégnation du support en deux étapes selon les méthodes d'imprégnation classiques (c'est-à dire sans utilisation d'une suspension colloïdale pour le Pd). Le catalyseur obtenu comprend une croûte relativement épaisse mais présentant un rapport de proximité RP dans une gamme de 0.5 à 2.

Une solution de nitrate de palladium est préparée sous agitation à 25°C par dilution de 2,7 g d'une solution de nitrate de palladium Pd(NO$_3$)$_2$ contenant 8,5 % poids de palladium Pd avec environ 50ml d'eau déminéralisée. Cette solution est ensuite imprégnée sur 80g d'une alumine dont la surface spécifique est de 71 m$^2$/g mise en forme sous forme de billes. Le solide obtenu est séché sous air 2 h à 120°C. Le catalyseur est ensuite calciné sous un flux d'air 2 h à 450°C.

Le solide obtenu est immergé dans 500ml d'une solution 2,3g/l d'acide formique. La solution est agitée pendant 2h à 25°C. Le solide est ensuite filtré et lavé en trois fois avec 500ml d'eau. Le solide réduit est immergé dans 500ml d'une solution 0,4g/l de nitrate d'argent à 30°C pendant5h. Le solide obtenu est filtré, lavé en trois fois avec 500ml d'eau. Le solide obtenu est séché sous air 2 h à 120°C. Le catalyseur est ensuite calciné sous un flux d'air 2 h à 570°C.

Le catalyseur C3 contient 0,28%poids de palladium et 0,17% poids d'argent.

La caractérisation du catalyseur par microsonde de Castaing montre que 80% du Pd est réparti sur une croûte d'épaisseur environ 200 $\mu$m. 80% de l'argent est réparti sur une croûte d'épaisseur environ 120 $\mu$m. La dispersion apparente du palladium du catalyseur C3 est de 20%. Le facteur de proximité RP est de 0,92.

**Exemple 4 :** préparation d'un catalyseur C4 non conforme à l'invention

Cet exemple montre la préparation d'un catalyseur non conforme à l'invention comprenant l'imprégnation du support en deux étapes selon les méthodes d'imprégnation classiques (c'est-à dire sans utilisation d'une suspension colloïdale pour le Pd). Le catalyseur obtenu comprend une croûte relativement fine et présente un rapport de proximité RP en dehors de la gamme de 0.5 à 2.

Une solution de nitrate de palladium est préparée sous agitation à 25°C par dilution de 2,7 g d'une solution de nitrate de palladium Pd(NO$_3$)$_2$ contenant 8,5 % poids de palladium Pd avec environ 50ml d'eau. Cette solution est ensuite imprégnée sur 80g d'une alumine dont la surface spécifique est de 71 m$^2$/g. Le solide obtenu est séché sous air 2 h à 120°C. Le catalyseur est ensuite calciné sous un flux d'air 2 h à 450°C.

Une solution de nitrate d'argent est préparée sous agitation à 25°C par dilution de 0,24g du sel métallique dans de l'eau déminéralisée à un volume qui correspond au volume poreux du support alumine. Le solide obtenu est séché sous air 2 h à 120°C. Le catalyseur est ensuite calciné sous un flux d'air 2 h à 450°C.

Le catalyseur C4 contient 0,29%poids de palladium et 0,2%poids d'argent.

La caractérisation du catalyseur C4 par microsonde de Castaing montre que 80% du Pd est réparti sur une croûte d'épaisseur environ 190 $\mu$m. 80% de l'argent est réparti sur une croûte d'épaisseur environ 40 $\mu$m. La dispersion apparente du palladium du catalyseur C4 est de 24%. Le facteur de proximité RP est de 0,30.

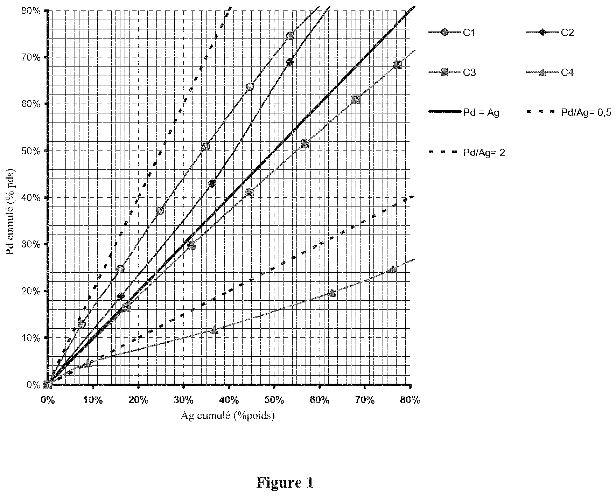

La représentation graphique du rapport RP pour les catalyseurs C1 à C4 est représentée en Figure 1. Les catalyseurs C1, C2 et C3 ont un RP compris entre 0,5 et 2 dans la croute où 80% poids des métaux Pd et Ag sont concentrés. Les deux métaux Pd et Ag sont donc localement bien associés dans la croûte. Le catalyseur C4 présente un RP en dehors de la gamme définie.

## Exemple 5 : Préparation d'un catalyseur C5 non conforme à l'invention

Cet exemple montre l'influence de la surface spécifique du support pour un catalyseur préparé selon l'invention comprenant l'imprégnation du support en deux étapes selon le procédé de l'invention, c'est-à-dire par utilisation d'une solution colloïdale pour le Pd. Le catalyseur obtenu comprend une croûte relativement épaisse et présente un rapport de proximité RP dans une gamme de 0.5 à 2.

Une suspension colloïdale d'oxyde de Pd est préparée sous agitation à 25°C par dilution de 1,8 g d'une solution de nitrate de palladium Pd(NO$_3$)$_2$ contenant 8,5 % poids de palladium Pd avec environ 45 ml d'eau déminéralisée, puis ajout d'environ 10 ml d'une solution de soude pour arriver à un pH de 2,4. La suspension est ensuite diluée avec de l'eau déminéralisée à un volume qui correspond au volume poreux du support alumine. Cette solution est ensuite imprégnée sur 80g d'une alumine dont la surface spécifique est de 5m$^2$/g mise en forme sous forme de billes. Une étape de maturation du support imprégné avant séchage d'une durée de 20 h est effectuée sous air en milieu confiné et humide. Le solide obtenu est séché sous air pendant 2 h à 120°C. Le catalyseur est ensuite calciné sous unflux d'air 2 h à 450°C.

Le solide obtenu est immergé dans 500ml d'une solution 1,4g/l d'acide formique. La solution est agitée pendant 2h à 25°C.Le solide est ensuite filtré et lavé en trois fois avec 500ml d'eau. Le solide réduit est alors immergé dans 500ml d'une solution à 0,23 g/L de nitrate d'argent à 30°C pendant 5h. Le solide obtenu est filtré, lavé en trois fois avec 500ml d'eau puis séché 3h à 120°C et calciné 2h à 570°C.

Le catalyseur C5 ainsi préparé contient 0,17%poids de palladium et 0,10% poids d'argent.

La caractérisation du catalyseur C5 par microsonde de Castaing montre que 80% du Pd est réparti sur une croûte d'épaisseur environ 180$\mu$m. 80% de l'argent est réparti dans croûte d'épaisseur environ 150 $\mu$m. Le sodium est réparti de façon homogène avec un coefficient de répartition R(Na)=0,92. La dispersion apparente du palladium du catalyseur C5 est de 24%. Le facteur de proximité RP est de 1,10.

## Exemple 6 : Utilisation des catalyseurs C1, C2, C3, C4 et C5 pour l'hydrogénation sélective de la coupe C3 de vapocraquage

Une charge comprenant 92,47% poids de propylène, 4,12%poids de propane, 1,18%poids de méthyl acétylène (MA), 1,63% poids de propadiène (PD) est traitée avec les catalyseurs C1, C2, C3, C4 et C5. Avant réaction les catalyseurs d'hydrogénation sélective sont activés sous un flux d'hydrogène à 160°C pendant 2h. 25ml de catalyseur sont placés dans un réacteur tubulaire en mode up flow. La pression est maintenue à 30 bar et la température à 27°C. Une vitesse horaire volumique (vvh) de 50h$^{-1}$ est appliquée. Le rapport molaire H$_2$/MPAD varie entre 0,5 et 4,5 mol/mol. La composition de la charge et de l'effluent est mesurée en continu en sortie du réacteur par chromatographie en phase gaz. Les performances sont exprimées en [C3$^=$effluent - C3$^=$charge] / [MAPD$_{effluent}$ - MAPD$_{charge}$] qui représente la sélectivité du catalyseur en fonction de la teneur résiduelle en MAPD.

Tableau 1

| Sélectivité [C3$^=$effluent - C3$^=$charge] / [MAPD$_{effluent}$ - MAPD$_{charge}$] en hydrogénation d'une coupe C3 de vapocraquage pour une teneur résiduelle en MAPD de 25ppm | | |
|---|---|---|
| Catalyseur | Sélectivité | RP |
| C1 | 51 | 1,40 |
| C2 | 60 | 1,27 |

(suite)

| Sélectivité [C3=effluent - C3=charge] / [MAPD$_{effluent}$ - MAPD$_{charge}$] en hydrogénation d'une coupe C3 de vapocraquage pour une teneur résiduelle en MAPD de 25ppm | | |
|---|---|---|
| Catalyseur | Sélectivité | RP |
| C3 | 30 | 0,92 |
| C4 | 2 | 0,30 |
| C5 | 28 | 1,10 |

Les catalyseurs ayant une croûte de palladium/argent fine (<120 microns), et un RP compris entre 0,5 et 2, sur un support ayant une surface spécifique supérieure à 65 m$^2$/g permettent d'améliorer la sélectivité de façon extrêmement significative.

**Revendications**

1. Procédé de préparation d'un catalyseur comprenant un grain de support poreux sur lequel est déposé du palladium et de l'argent et au moins un métal sélectionné dans le groupe constitué par les alcalins et les alcalino-terreux, le support poreux comprenant au moins un oxyde réfractaire sélectionné dans le groupe constitué par la silice, l'alumine et la silice-alumine, la surface spécifique du support poreux étant comprise entre 10 et 150 m$^2$/g, la teneur en palladium dans le catalyseur étant comprise entre 0,05 et 0,6 % poids, la teneur en argent dans le catalyseur étant comprise entre 0,02 et 3 % poids, au moins 80 % poids de palladium étant réparti dans une croûte à la périphérie du support, l'épaisseur de ladite croûte étant comprise entre 10 et 160 $\mu$m, au moins 80 % poids de l'argent étant réparti dans une croûte à la périphérie du support, l'épaisseur de ladite croûte étant comprise entre 10 et 160 $\mu$m, la teneur locale en palladium à chaque point le long du diamètre du grain ayant la même évolution que la teneur locale en argent, la somme des teneurs en métaux alcalins et/ou alcalino-terreux étant comprise entre 0,02 et 5 % poids, ledit procédé comprenant les étapes suivantes:

   - une étape dans laquelle on introduit le palladium sur le support, dite étape 1, comprenant les étapes suivantes :

      - une étape 1a), dans laquelle on prépare dans un appareillage une suspension colloïdale d'oxyde de palladium ou d'hydroxyde de palladium en phase aqueuse par mélange d'une solution aqueuse 1 comprenant au moins un hydroxyde sélectionné dans le groupe constitué par les hydroxydes d'alcalins et les hydroxydes d'alcalino-terreux et d'une solution aqueuse 2 comprenant au moins un précurseur du palladium, la solution 2 puis la solution 1 étant versées dans l'appareillage ou les solutions 1 et 2 étant versées simultanément dans l'appareillage,
      - une étape 1b), dans laquelle on imprègne ladite suspension colloïdale sur ledit grain de support poreux ayant une surface spécifique comprise entre 10 et 150 m$^2$/g,
      - une étape 1c), dans laquelle on mature ledit support imprégné obtenu à l'étape 1b),
      - une étape 1d), dans laquelle on sèche le catalyseur obtenu à l'étape 1c),
      - une étape 1e), dans laquelle on calcine le catalyseur obtenu à l'étape 1d),

   - puis une étape dans laquelle on introduit l'argent, dite étape 2, comprenant les étapes suivantes :

      - une étape 2a), dans laquelle on réduit le catalyseur préparé selon l'étape 1 par mise en contact avec une solution aqueuse comprenant au moins un agent réducteur en phase liquide,
      - une étape 2b), dans laquelle on filtre le catalyseur obtenu à l'étape 2a),
      - une étape 2c), dans laquelle on imprègne le catalyseur préparé selon l'étape 2b) par mise en contact sous agitation avec une solution aqueuse comprenant un sel précurseur de l'argent,
      - une étape 2d), dans laquelle on filtre le catalyseur obtenu à l'étape 2c),
      - une étape 2e), dans laquelle on sèche le catalyseur obtenu à l'étape 2d),
      - une étape 2f), dans laquelle on calcine le catalyseur obtenu à l'étape 2e), de préférence entre 450°C et 700°C.

2. Procédé de préparation selon la revendication 1, dans lequel dans l'étape 1a), le précurseur du palladium est sélectionné dans le groupe constitué par le chlorure de palladium, le nitrate de palladium et le sulfate de palladium.

**3.** Procédé de préparation selon les revendications 1 ou 2, dans lequel dans l'étape 2c), le précurseur de l'argent est sélectionné dans le groupe constitué par le nitrate d'argent, l'acétate d'argent, le citrate d'argent, le chlorure d'argent et l'oxalate d'argent.

**4.** Procédé de préparation selon l'une des revendications 1 à 3, dans lequel dans l'étape 2a), l'agent réducteur est choisi parmi l'acide formique, l'acide citrique, l'acide ascorbique, l'acide oxalique, le formiate de sodium, l'acétate de sodium, le borohydrure de sodium, le formaldéhyde, le dextrose, l'hydrazine et l'hydrogène.

**5.** Procédé de préparation d'un catalyseur selon les revendications 1 à 4, dans lequel l'évolution de la teneur en palladium et de la teneur en argent s'exprime par un rapport de proximité RP compris entre 0.5 et 2, le dit rapport de proximité RP étant défini par la formule suivante :

$$\text{Rapport de proximité} \qquad RP(y) = \frac{Q(y)Pd / Q(r)Pd}{Q(y)Ag / Q(r)Ag}$$

avec :

Q (y) Pd = somme des concentrations en palladium entre le bord du grain et une distance y du bord du grain catalytique (%pds)
Q (y) Ag = somme des concentrations en argent entre le bord du grain et une distance y du bord du grain catalytique (%pds)
Q (r) Pd = teneur en palladium totale du grain catalytique (%pds) Q (r) Ag= teneur en argent totale du grain catalytique (%pds).

**6.** Procédé de préparation d'un catalyseur selon les revendications 1 à 5, dans lequel ledit métal alcalin et/ou alcalino-terreux est réparti de manière homogène à travers le grain de support avec un coefficient R compris entre 0,8 et 1,2, ledit coefficient R étant défini par la formule suivante :

$$R = \int_{-r}^{r} c(x)x^2 dx \Big/ \frac{r^2}{3} \int_{-r}^{r} c(x)dx$$

avec le profil de répartition $c(x)$ pour $x \in [-r;+r]$ obtenu par la microsonde de Castaing, c la concentration locale de l'élément alcalin ou/alcalino-terreux, r le rayon de la bille, et X la position du point d'analyse le long du diamètre du grain par rapport au centre de ce grain.

**7.** Procédé de préparation d'un catalyseur selon l'une des revendications 1 à 6, dans lequel ledit support poreux est de l'alumine.

**8.** Procédé de préparation d'un catalyseur selon l'une des revendications 1 à 7, dans lequel ledit grain de support poreux se présente sous forme de billes ou d'extrudés.

**9.** Procédé de préparation d'un catalyseur selon l'une des revendications 1 à 8, dans lequel ledit métal alcalin est le sodium.

**10.** Procédé de préparation d'un catalyseur selon l'une des revendications 1 à 9, dans lequel la surface spécifique dudit support poreux est comprise entre 65 et 150 $m^2/g$ et dans lequel la teneur en palladium dans le catalyseur est comprise entre 0,05 et 0,4 % poids, la teneur en argent dans le catalyseur est comprise entre 0,05 et 0,3 % poids, au moins 80 % poids de palladium est réparti dans une croûte à la périphérie du support, l'épaisseur de ladite croûte est comprise entre 10 et 110 $\mu$m, au moins 80 % poids de l'argent est réparti dans une croûte à la périphérie du support, l'épaisseur de ladite croûte est comprise entre 10 et 110 $\mu$m.

**Patentansprüche**

**1.** Verfahren zur Herstellung eines Katalysators, umfassend ein Korn eines porösen Trägers, auf dem Palladium und Silber abgeschieden sind, und mindestens ein Metall, ausgewählt aus der Gruppe bestehend aus Alkali- und Erdalkalimetallen, wobei der poröse Träger mindestens ein hitzebeständiges Oxid umfasst, ausgewählt aus der Gruppe bestehend aus Siliciumdioxid, Aluminiumoxid und Siliciumdioxid-Aluminiumoxid, wobei die spezifische Oberfläche des porösen Trägers im Bereich zwischen 10 und 150 m$^2$/g liegt, wobei der Palladiumgehalt in dem Katalysator im Bereich von 0,05 bis 0,6 Gew.-% liegt, wobei der Silbergehalt in dem Katalysator im Bereich von 0,02 bis 3 Gew.-% liegt, wobei mindestens 80 Gew.-% Palladium in einer Kruste am Rand des Trägers verteilt sind, wobei die Dicke der Kruste im Bereich von 10 und 160 $\mu$m liegt, wobei mindestens 80 Gew.-% Silber in einer Kruste am Rand des Trägers verteilt sind, wobei die Dicke der Kruste im Bereich von 10 und 160 $\mu$m liegt, wobei der lokale Palladiumgehalt an jedem Punkt entlang des Korndurchmessers den gleichen Verlauf aufweist wie der lokale Silbergehalt, wobei die Summe der Alkali- und/oder Erdalkalimetallgehalte im Bereich von 0,02 bis 5 Gew.-% liegt, wobei das Verfahren die folgenden Schritte umfasst:

- einen Schritt zum Einführen des Palladiums in den Träger, genannt Schritt 1, der die folgenden Schritte umfasst:

- einen Schritt 1a), wobei in einer Vorrichtung eine kolloidale Suspension aus Palladiumoxid oder Palladiumhydroxid in einer wässrigen Phase durch Mischen einer wässrigen Lösung 1, umfassend mindestens ein Hydroxid, ausgewählt aus der Gruppe bestehend aus Alkalihydroxiden und Erdalkalihydroxiden, und einer wässrigen Lösung 2, umfassend mindestens eine Palladiumvorstufe, hergestellt wird, wobei Lösung 2, dann Lösung 1 in die Vorrichtung gegossen werden oder die Lösungen 1 und 2 gleichzeitig in die Vorrichtung gegossen werden,
- einen Schritt 1b), wobei die kolloidale Suspension auf das Korn des porösen Trägers imprägniert wird, der eine spezifische Oberfläche im Bereich von 10 bis 150 m$^2$/g aufweist,
- einen Schritt 1c), wobei der in Schritt 1b) erhaltene imprägnierte Träger gereift wird,
- einen Schritt 1d), wobei der in Schritt 1c) erhaltene Katalysator getrocknet wird,
- einen Schritt 1e), wobei der in Schritt 1d) erhaltene Katalysator kalziniert wird,

- dann einen Schritt zum Einführen des Silbers, genannt Schritt 2, der die folgenden Schritte umfasst:

- einen Schritt 2a), wobei der gemäß Schritt 1 hergestellte Katalysator durch Inkontaktbringen mit einer wässrigen Lösung, umfassend mindestens ein Flüssigphasen-Reduktionsmittel, reduziert wird,
- einen Schritt 2b), wobei der in Schritt 2a) erhaltene Katalysator filtriert wird,
- einen Schritt 2c), wobei der gemäß Schritt 2b) hergestellte Katalysator durch Inkontaktbringen unter Rühren mit einer wässrigen Lösung, umfassend ein Silbervorstufensalz, imprägniert wird,
- einen Schritt 2d), wobei der in Schritt 2c) erhaltene Katalysator filtriert wird,
- einen Schritt 2e), wobei der in Schritt 2d) erhaltene Katalysator getrocknet wird,
- einen Schritt 2f), wobei der in Schritt 2e) erhaltene Katalysator vorzugsweise zwischen 450 °C und 700 °C kalziniert wird.

2. Herstellungsverfahren nach Anspruch 1, wobei in Schritt 1a) die Palladiumvorstufe ausgewählt ist aus der Gruppe bestehend aus Palladiumchlorid, Palladiumnitrat und Palladiumsulfat.

3. Herstellungsverfahren nach den Ansprüchen 1 oder 2, wobei in Schritt 2c) die Silbervorstufe ausgewählt ist aus der Gruppe bestehend aus Silbernitrat, Silberacetat, Silbercitrat, Silberchlorid und Silberoxalat.

4. Herstellungsverfahren nach einem der Ansprüche 1 bis 3, wobei in Schritt 2a) das Reduktionsmittel ausgewählt ist aus Ameisensäure, Citronensäure, Ascorbinsäure, Oxalsäure, Natriumformiat, Natriumacetat, Natriumborhydrid, Formaldehyd, Dextrose, Hydrazin und Wasserstoff.

5. Verfahren zur Herstellung eines Katalysators nach den Ansprüchen 1 bis 4, wobei der Verlauf des Palladiumgehalts und des Silbergehalts durch ein Proximitätsverhältnis RP im Bereich 0,5 bis 2 ausgedrückt wird, wobei das Proximitätsverhältnis RP durch die folgende Formel definiert ist:

Proximitätsverhältnis
$$RP(y) = \frac{Q(y)Pd \ / \ Q(r)Pd}{Q(y)Ag \ / \ Q(r)Ag}$$

wobei:

Q(y)Pd = Q (y) Pd = Summe der Palladiumkonzentrationen zwischen dem Rand des Korns und einem Abstand y vom Rand des katalytischen Korns (Gew.-%)

Q(y)Ag = Q (y) Pd = Summe der Silberkonzentrationen zwischen dem Rand des Korns und einem Abstand y vom Rand des katalytischen Korns (Gew.-%)

Q(r)Pd = Gesamtpalladiumgehalt des katalytischen Korns (Gew. %)

Q(r)Ag = Gesamtsilbergehalt des katalytischen Korns (Gew. %)

6. Verfahren zur Herstellung eines Katalysators nach den Ansprüchen 1 bis 5, wobei das das Alkali- und/oder Erdalkalimetall über das Trägerkorn mit einem Koeffizienten R im Bereich von 0,8 bis 1,2 homogen verteilt ist, wobei der Koeffizient R durch die folgende Formel definiert ist:

$$R = \int_{-r}^{r} c(x)x^2 dx \Big/ \frac{r^2}{3} \int_{-r}^{r} c(x)dx$$

wobei das Verteilungsprofil c(x) für $x \in$ [-r;+r] mit der Castaing-Mikrosonde erhalten wird, c die lokale Konzentration des Alkali- und/oder Erdalkalielement ist, r der Perlenradius ist, und X die Position des Analysepunkts entlang des Durchmessers des Korns in Bezug auf das Zentrum dieses Korns ist.

7. Verfahren zur Herstellung eines Katalysators nach einem der Ansprüche 1 bis 6, wobei der poröse Träger Aluminiumoxid ist.

8. Verfahren zur Herstellung eines Katalysators nach einem der Ansprüche 1 bis 7, wobei das Korn des porösen Trägers die Form von Perlen oder Extrudaten aufweist.

9. Verfahren zur Herstellung eines Katalysators nach einem der Ansprüche 1 bis 8, wobei das Alkalimetall Natrium ist.

10. Verfahren zur Herstellung eines Katalysators nach einem der Ansprüche 1 bis 9, wobei die spezifische Oberfläche des porösen Trägers im Bereich von 65 bis 150 m$^2$/g liegt und wobei der Palladiumgehalt in dem Katalysator im Bereich von 0,05 bis 0,4 Gew.-% liegt, wobei der Silbergehalt in dem Katalysator im Bereich von 0,05 bis 0,3 Gew.-% liegt, wobei mindestens 80 Gew.-% Palladium in einer Kruste am Rand des Trägers verteilt sind, wobei die Dicke der Kruste im Bereich von 10 und 110 $\mu$m liegt, wobei mindestens 80 Gew.-% Silber in einer Kruste am Rand des Trägers verteilt sind, wobei die Dicke der Kruste im Bereich von 10 und 110 $\mu$m liegt.

## Claims

1. A process for preparing a catalyst comprising a porous support grain on which are deposited palladium and silver, and at least one metal selected from the group consisting of the alkalis and the alkaline earths, the porous support comprising at least one refractory oxide selected from the group consisting of silica, alumina and silica-alumina, the specific surface area of the porous support being within the range 10 to 150 m$^2$/g, the palladium content of the catalyst within the range 0.05 to 0.6 wt.%, the silver content of the catalyst within the range 0.02 to 3 wt.%, at least 80 wt.% of the palladium being distributed in a crust at the periphery of the support, the thickness of the said crust being within the range 10 to 160 $\mu$m, at least 80 wt.% of the silver being distributed in a crust at the periphery of the support, the thickness of the said crust being within the range 10 to 160 $\mu$m, the local content of palladium at each point along the diameter of the grain following the same course as the local content of silver, the sum of the contents of alkali and/or alkaline earth metals being within the range 0.02 to 5 wt.%.

  - a step wherein the palladium is introduced onto the support, referred to as step 1, comprising the following steps :

    - a step 1a) wherein, in an apparatus, a colloidal suspension of palladium oxide or palladium hydroxide is prepared in an aqueous phase by mixing an aqueous solution 1 comprising at least one hydroxide selected from the group consisting of alkali hydroxides and alkaline-earth hydroxides and an aqueous solution 2 comprising at least one palladium precursor, the solution 2 then the solution 1 being poured into the apparatus or solutions 1 and 2 being poured simultaneously into the apparatus,
    - a step 1b) wherein the said colloidal suspension is impregnated onto the said porous support grain having a specific surface area within the range 10 to 150 m2/g,
- a step 1c) wherein the said impregnated support obtained in step 1b) is matured,
- a step 1d) wherein the catalyst obtained in step 1c) is dried,
- a step 1e) wherein the catalyst obtained in step 1d) is calcined,

- then a step wherein the silver is introduced, referred to as step 2, comprising the following steps :

- a step 2a), wherein the catalyst prepared in accordance with step 1 is reduced by placing it in contact with an aqueous solution comprising at least one liquid phase reducing agent,
- a step 2b), wherein the catalyst obtained in step 2a) is filtered,
- a step 2c), wherein the catalyst prepared in step 2b) is impregated by placing it in contact, under agitation, with an aqueous solution comprising a silver precursor salt,
- a step 2d), wherein the catalyst obtained in step 2c) is filtered,
- a step 2e), wherein the catalyst obtained in step 2d) is dried,
- a step 2f), wherein the catalyst obtained in step 2e) is calcined, preferably at 450°C to 700°C.

2. A process for preparing the catalyst according to claim 1, wherein, in step 1a), the palladium precursor is selected from the group consisting of palladium chloride, palladium nitrate, and palladium sulphate.

3. A process for preparing the catalyst according to either claim 1 or claim 2, wherein, in step 2c), the silver precursor is selected from the group consisting of silver nitrate, silver acetate, silver citrate, silver chloride, and silver oxalate.

4. A process for preparing the catalyst according to any one of claims 1 to 3, wherein, in step 2a), the reducing agent is selected from formic acid, citric acid, ascorbic acid, oxalic acid, sodium formiate, sodium acetate, sodium borohydride, formaldehyde, dextrose, hydrazine and hydrogen.

5. A process for preparing the catalyst according to any one of claims 1 to 4, wherein the course of the content of palladium and the content of silver may be expressed by a proximity ratio PR within the range 0.5 to 2, the said proximity ratio being defined by the following formula:

Proximity ratio:
$$PR(y) = \frac{Q(y)Pd / Q(r)Pd}{Q(y)Ag / Q(r)Ag}$$

where :

Q (y) Pd = Sum of the palladium concentrations between the edge of the catalytic grain and a distance y from the edge of the grain (wt.%)
Q (y) Ag = Sum of the silver concentrations between the edge of the catalytic grain and a distance y from the edge of the grain (wt.%)
Q (r) Pd = Total palladium content of the catalytic grain (wt.%) Q (r) Ag = Total silver content of the catalytic grain (wt.%).

6. A process for preparing the catalyst according to any one of claims 1 to 5, wherein the said alkali and/or alkaline-earth metal is homogeneously distributed through the support grain with a coefficient R within the range 0.8 to 1.2, the said coefficient R being defined by the following formula.

$$R = \int_{-r}^{r} c(x)x^2 dx \bigg/ \frac{r^2}{3} \int_{-r}^{r} c(x) dx$$

where the distribution profile $c(x)$ for $x \in [-r, +r]$ is obtained with a Castaing microprobe, c being the local concentration locale of the alkali and/or alkaline-earth element, r the radius of the bead, and x the position of the analysis point along the diameter of the grain relative to the centre of this grain.

7. A process for preparing the catalyst according to any one of claims 1 to 6, wherein the porous support is alumina.

8. A process for preparing the catalyst according to any one of claims 1 to 7, wherein the porous support grain is in the form of beads or extrudates.

9. A process for preparing the catalyst according to any one of claims 1 to 8, wherein the said alkali metal is sodium.

10. A process for preparing the catalyst according to any one of claims 1 to 9, wherein the specific surface area of the said porous support is within the range 65 to 150 $m^2$/g and wherein the content of palladium in the catalyst is within the range 0.05 to 0.4 wt.%, the silver content of the catalyst is within the range 0.05 to 0.3 wt.%, at least 80 wt.% of the palladium is distributed within a crust at the periphery of the support, the thickness of the said crust is within the range 10 to 110 $\mu$m, at least 80 wt.% of the silver is distributed within a crust at the periphery of the support, and the thickness of the said crust is within the range 10 to 110 $\mu$m.

**Figure 1**

## RÉFÉRENCES CITÉES DANS LA DESCRIPTION

**Documents brevets cités dans la description**

- FR 2922784
- FR 2882531
- US 2010217052 A
- EP 0780155 A

**Littérature non-brevet citée dans la description**

- **M. BOUDART ; W.C. CHENG.** *J. Catal.,* 1987, vol. 106, 134
- **S. HUB ; L. HILAIRE ; R. TOUROUDE.** *Appl. Catal.,* 1992, vol. 36, 307
- **V. PONEC ; G.C. BOND.** Catalysis by Metal and Alloys. Elsevier, 1995
- Analyse physico-chimique des catalyseurs industriels. 2001
- **R. VAN HARDEVELD ; F. HARTOG.** *Surf. Sci.,* 1969, vol. 15, 189